# EUROPÄISCHE PATENTSCHRIFT

(11) **EP 2 895 065 B1**
(45) Veröffentlichungstag und Bekanntmachung des Hinweises auf die Patenterteilung: **05.10.2016**
(21) Anmeldenummer: 13752619.0
(22) Anmeldetag: 16.08.2013
(51) Int. Cl.: A61B 5/05, A61B 5/055, G01R 33/12

(54) **VORRICHTUNG ZUR ERZEUGUNG EINES MAGNETFELDVERLAUFES, WELCHER SOWOHL DEN ANFORDERUNGEN FÜR MPI ALS AUCH FÜR MRI GENÜGT**
DEVICE FOR GENERATING A MAGNETIC FIELD PROFILE WHICH MEETS THE REQUIREMENTS FOR MPI AND FOR MRI
DISPOSITIF GÉNÉRATEUR DE GRADIENT DE CHAMP MAGNÉTIQUE RÉPONDANT À LA FOIS AUX EXIGENCES DU CONTRÔLE MAGNÉTOSCOPIQUE ET À CELLES DE L'IMAGERIE PAR RÉSONANCE MAGNÉTIQUE

(30) Priorität: 14.09.2012 DE 102012216357
(43) Veröffentlichungstag der Anmeldung: 22.07.2015
(73) Patentinhaber: Bruker BioSpin MRI GmbH, 76275 Ettlingen (DE)
(72) Erfinder: HEIDENREICH, Michael, 76228 Karlsruhe (DE); FRANKE, Jochen, 76185 Karlsruhe (DE); NIEMANN, Volker, 75228 Ispringen (DE); PIETIG, Rainer, 76316 Malsch (DE)
(74) Vertreter: Kohler Schmid Möbus Patentanwälte
(86) Internationale Anmeldenummer: PCT/EP2013/067149
(87) Internationale Veröffentlichungsnummer: WO 2014/040822

(56) Entgegenhaltungen:
- WO-A1-2012/077015
- JP-A- 2009 195 614
- WEIZENECKER J ET AL: "LETTER TO THE EDITOR; Three-dimensional real-time in vivo magnetic particle imaging", PHYSICS IN MEDICINE AND BIOLOGY, INSTITUTE OF PHYSICS PUBLISHING, BRISTOL GB, Bd. 54, Nr. 5, 7. März 2009 (2009-03-07), Seiten L1-L10, XP020149861, ISSN: 0031-9155, DOI: 10.1088/0031-9155/54/5/L01 in der Anmeldung erwähnt
- KNOPP T ET AL: "FAST TRACK COMMUNICATION; Field-free line formation in a magnetic field", JOURNAL OF PHYSICS A: MATHEMATICAL AND THEORETICAL, JOURNAL OF PHYSICS A: MATHEMATICAL AND THEORETICAL, BR, Bd. 43, Nr. 1, 8. Januar 2010 (2010-01-08) , Seite 12002, XP020170831, ISSN: 1751-8121
- FRANKE J ET AL: "First hybrid MPI-MRI imaging system as integrated design for mice and rats: Description of the instrumentation setup", MAGNETIC PARTICLE IMAGING (IWMPI), 2013 INTERNATIONAL WORKSHOP ON, IEEE, 23. März 2013 (2013-03-23), Seite 1, XP032424305, DOI: 10.1109/IWMPI.2013.6528363 ISBN: 978-1-4673-5520-9

## Beschreibung

Die Erfindung betrifft eine Vorrichtung zur abwechselnden Untersuchung eines Messobjektes mittels MPI (="Magnetic Particle Imaging") und mittels MRI (="Magnetic Resonance Imaging") innerhalb eines aus zwei Magnetfelderzeugenden Elementen aufgebauten Magnetsystems, wobei das Magnetsystem einen ersten Untersuchungsbereich für den MRI-Betrieb aufweist, in welchem ein homogenes Magnetfeld erzeugt wird, und wobei das Magnetsystem einen zweiten Untersuchungsbereich für den MPI-Betrieb aufweist, in welchem ein räumlich stark variierendes Magnetfeldprofil erzeugt wird, dessen Feldvektoren an allen Raumpunkten bezüglich Richtung und/oder Betrag unterschiedlich sind und an einem Raumpunkt den Feldbetrag Null umfassen.

Eine solche Vorrichtung ist bekannt aus Referenz [3] (Weizenecker et al., 2009).

In den letzten Jahrzehnten sind zahlreiche tomographische bildgebende Verfahren wie zum Beispiel die Computertomographie (CT) von Hounsfield im Jahre 1969, die Magnetresonanztomographie (MRI) von Lauterbur und Mansfield im Jahre 1973 oder die Positronen-Emissions-Tomographie (PET) von Ter-Pogossian und Phelps im Jahre 1975 erfunden worden. Durch stetige Hardware-Sequenz- und/oder Rekonstruktionsalgorithmen-Weiterentwicklung nehmen in der heutigen medizinischen Diagnostik bildgebende Verfahren einen immer größeren Stellenwert ein. Mittels Kombination einzelner bildgebender Verfahren zu sogenannten Hybridsystemen (z.B. PET-CT seit 2001 und MRI-PET seit 2010 im klinischen Betrieb) konnte die diagnostische Aussagekraft durch bildgebende Verfahren weiter erhöht werden. Allen Hybridsystemen liegt zu Grunde, dass komplementäre informationen der einzelnen Modalitäten synergetisch kombiniert und/oder graphisch überlagert werden. So werden beispielsweise die CT-Daten eines PET-CT Hybridsystem für morphologische Informationen sowie zur Schwächungskorrektur der PET-Daten herangezogen.

Im Jahre 2001 wurde mit Magnetic Particle Imaging (MPI) ein weiteres tomographisches bildgebendes Verfahren von Gleich und Weizenäcker (DE10151778A1) erfunden. Dieses junge und sich schnell entwickelnde volumetrische bildgebende Verfahren dient der Detektion der räumlichen Verteilung von applizierten superparamagnetischen Nanopartikeln (SPIO). Dieses Verfahren bietet ein räumliches sowie ein hohes zeitliches Auflösungsvermögen (siehe Referenzen [1-3]).

Das grundlegende Prinzip für MPI beruht auf einer Anregung der Nanopartikel mittels eines zeitlich sich ändernden Magnetfeldes - dem sogenannten "Drive Field" (DF) - mit einer Anregungsfrequenz f₀. Durch die nichtlineare Magnetisierungskurve der SPIO's entstehen als Partikelantwort Harmonische von f₀, welche mittels Empfangsspulen detektiert und zur Bildrekonstruktion genutzt werden. Da Gewebe eine vernachlässigbar kleine nichtlineare Antwort auf die Anregungsfrequenz f₀ aufweist, bietet dieses Verfahren einen hohen Kontrast durch die Akquisition von lediglich der Partikelantwort. Eine räumliche Kodierung wird auf den Effekt gestützt, dass die Partikelmagnetisierung ab einer bestimmten magnetischen Feldstärke in Sättigung geht. Durch die magnetische Anregung mit der Frequenz f0 ändert sich die Magnetisierung der gesättigten SPIO's nur minimal und tragen demnach nicht/kaum zur Partikelantwort bei. Um diesen Effekt der Saturierung zu nutzen, wird ein statischer Magnetfeldgradient - das sogenannte "Selektionsfeld" (SF) - mit einem feldfreien Punkt (FFP) erzeugt. Ausgehend von dem FFP steigt die magnetische Feldstärke in alle Raumrichtungen an.

Ein solcher Magnetfeldverlauf kann z.B. durch Permanentmagnete mit entgegengesetzter Magnetisierungsrichtung oder mittels eines Maxwell-Elektromagnetspulenpaares erzeugt werden. Durch den Saturierungseffekt werden nur Partikel in unmittelbarer Nähe zu dem FFP angeregt und steuern somit zur Partikelantwort bei. Die Ausdehnung des FFP's, und damit die Sensitivität des MPI Verfahrens, ist abhängig von der magnetischen Feldstärke bei welcher die Partikel in Sättigung gelangen sowie von der Gradientenstärke des SF, mit der das Magnetfeld ausgehend von den FFP ansteigt (siehe Referenzen [4, 5]). Um eine volumetrische Bildgebung zu erlauben, wird der FFP relativ zum Untersuchungsobjekt durch z.B. Superposition zusätzlicher Magnetfelder und/oder durch mechanische Bewegung des Untersuchungsobjektes gesteuert.

Das quantitative Verfahren MPI bietet durch seine hohe Sensitivität sowie sein hohes zeitliches Auflösungsvermögen erfolgsversprechende nichtinvasive Anwendungsmöglichkeiten im Bereich der molekularen und medizinischen Bildgebung wie zum Beispiel Zell-Verfolgung (cell tracking) oder Krebsdiagnostik sowie im Bereich der Herz-Kreislauf-Diagnostik und Blutgefäßbildgebund. Im Gegensatz zu anderen bildgebenden Verfahren wie zum Beispiel CT und MRI weisen die akquirierten MPI-Bilddatensätze heutzutage noch eine relative geringe räumliche Auflösung im Millimeterbereich auf. Diese Auflösungsbeschränkung ist durch die derzeit verfügbaren Nanopartikeln sowie den technisch realisierbaren Magnetfeldgradienten gegeben. Des Weiteren kann aus den Daten mit einer ausschließlich zu den applizierten Nanopartikeln hohen Sensitivität eine Aussage über die quantitative Verteilung der Nanopartikeln getroffen werden, welche jedoch nur eine begrenzte morphologische Information beinhaltet. Dies macht eine eindeutige Zuordnung der gemessenen Partikelverteilung zu ihrem morphologischen Entstehungsort extrem schwierig.

Andere volumetrische bildgebende Verfahren, wie zum Beispiel das klinisch seit langem genutzte Verfahren der MRI, sind bestens geeignet um hoch aufgelöste morphologische Informationen zu erfassen. Die MRI-Technik basiert auf einem starken homogenen Magnetfeld sowie elektromagnetischen Wechselfeldern im Radiofrequenzbereich, mit denen bestimmte Atomkerne des Untersuchungsobjektes resonant angeregt werden (siehe Referenz [6]). Die angeregten Atomkerne emittieren wiederum elektromagnetische Wechselfelder, welche elektrische Signale in die Empfangsspule induzieren. Durch Nutzung von mehreren Magnetfeldgradienten wird das Signal räumlich kodiert und kann durch geeignete Algorithmen rekonstruiert werden. MRI erlaubt nicht nur die Akquisition hoch aufgelöste anatomische Informationen mit vielfältigen Weichgewebekontrasten im Submillimeterbereich, sondern bietet weiter differenzierte Techniken, die den Zugriff auf viele physiologische Parameter wie zum Beispiel Wasserdiffusion oder Permeabilität erlauben. Bei Nutzung von MR spektroskopischer Bildgebung können zudem metabolische und biochemische Abläufe räumlich dargestellt werden. Im Gegensatz zu MPI ist die MRI-Technik ein relativ unsensitives und langsames bildgebendes Verfahren mit Akquisitionszeiten im Bereich von Sekunden bis Minuten.

Durch die einzigartigen Eigenschaften beider volumetrischen bildgebenden Modalitäten sind MPI und MRI in Bezug auf ihren Informationsgehalt weitestgehend komplementär. Superior diagnostische Aussagekraft kann durch Kombination beider Verfahren und die synergetische Nutzung ihrer Eigenschaften - die hohe Sensitivität sowie das zeitliche Auflösungsvermögen der MPI-Technik und die vielfältigen Weichgewebekontraste und somit ausgezeichnete morphologische Information der MRI-Technik - ermöglicht werden. Eine Überlagerung/Fusion beider komplementären Bilddatensätze wurde bisher nur mittels zwei separaten bzw. unabhängigen Modalitäten von MPI und MRI realisiert (siehe Referenz [3]), da derzeit weltweit noch kein integrales Kombinationsgerät (Hybridgerät) dieser beiden Modalitäten zur Verfügung steht.

Die Nutzung zweier separater Modalitäten birgt jedoch einige Schwierigkeiten. Hierzu zählt unter anderem die Ko-Registrierung beider Datensätze mit unterschiedlichen Bezugskoordinaten, welche durch weitgehend unvermeidbare Verschiebung und Deformation durch Umlagerung bzw. Transport des Untersuchungsobjektes von der einen Modalität zu der anderen erschwert wird. Der Intermodalitätentransport vermindert zudem eine direkte Korrelation beider Datensätze im Zeitbereich. Weitere logistische Probleme entstehen etwa bei Kleintierstudien, welche eine kontinuierliche Anästhesierung des Versuchstieres voraussetzt. Die Bereitstellung zweier stand-alone Modalitäten bedeutet auch zugleich einen hohen Kosten- und Platzaufwand. Eine Kombination beider Modalitäten in ein integral aufgebautes Hybridgerät wurde in JP-2009195614-A2 vorgestellt, wohingegen das hierbei beschriebene Hybridgerät zwei unterschiedliche Modi (MRI und MPI) besitzt, welche durch das An- bzw. Abschalten von Teilspulensystemen realisiert werden.

### Aufgabe der Erfindung

Demgegenüber ist es Aufgabe der vorliegenden Erfindung, eine gattungsgemäße Vorrichtung mit den eingangs definierten Merkmalen kostengünstig und mit möglichst einfachen technischen Mitteln derart zu verbessern, dass die oben beschriebenen Schwierigkeiten bei der Kombination beider Modalitäten in ein integral aufgebautes Hybridgerät gemindert bzw. vermieden werden, wobei der gesamte Platzbedarf für beide Modalitäten verringert und die Komplexität des integral aufgebauten Hybridsystems minimiert werden sollen und eine Umschaltung zwischen den beiden Modi in Bezug auf das Magnetfeldprofil entfällt.

### Kurze Beschreibung der Erfindung und bevorzugter Ausführungsformen

Diese Aufgabe wir durch eine Vorrichtung gemäß Patenanspruch 1 gelöst.

Mit der vorliegenden Erfindung werden mögliche Magnetsystemanordnungen vorgeschlagen, die sowohl der MRI- als auch der MPI-Bildgebung genügen. Die zwei getrennten Untersuchungsbereiche ermöglichen eine optimale Ausnutzung des jeweiligen Messvolumens. Der MPI-Teil der Vorrichtung kann beispielsweise bei einer bereits vorhandenen MRI-Anlage nachgerüstet werden.

Für ein integral aufgebautes Hybridgerät bestehend aus der MRI- und MPI-Modalität muss die Magnetsystemanordnung einen Magnetfeldverlauf generieren, der sowohl einen homogenen Feldbereich für die MR-Bildgebung beinhaltet als auch einen Feldbereich mit einem räumlich verlaufenden Feldgradienten inklusive eines Feldnulldurchganges. Die Feldregion, die den Feldbetrag Null beinhaltet, wird in der MPI-Technologie Feldfreier Punkt (FFP) genannt.

Weitere Varianten der Erfindung sowie vorteilhafte Eigenschaften und Ausgestaltungen sind als Ausführungsformen und Weiterbildungen der Erfindung in den Unteransprüchen beschrieben.

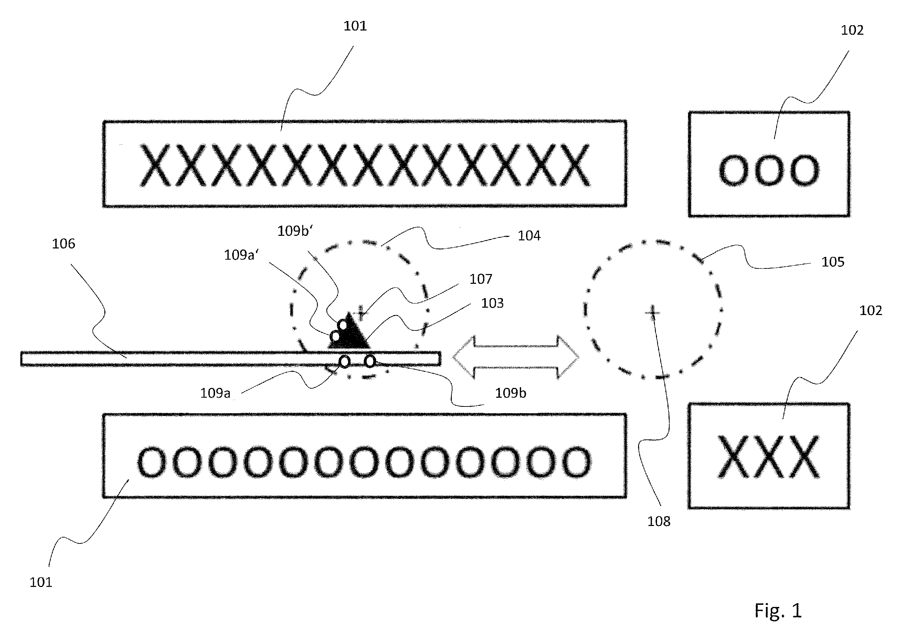

Besonders vorteilhaft sind Ausführungsformen der Erfindung, bei welchen beide Magnetfelderzeugenden Elemente des Magnetsystems in einem gemeinsamen Gehäuse angeordnet sind. Ein Hybridgerät bestehend aus der MRI und MPI-Modalität unterscheidet sich von zwei separaten bzw. stand-alone Modalitäten darin, dass das System integral aufgebaut ist, d.h. mindestens ein Magnetfelderzeugendes Element trägt signifikant zu dem Magnetfeldverlauf des Untersuchungsbereiches beider Modalitäten bei (siehe Fig. 1). Integral aufgebaute Hybridgeräte ermöglichen ferner eine gemeinsame Nutzung weitere Komponenten wie z.B. die Steuerungssoftware als auch Teilkomponenten der Sende- und/oder Empfangskette.

In den hier beschriebenen Aufbauten kann das Messobjekt mittels Transporteinheit (schematisch in Fig. 1 zu sehen) zwischen beiden Untersuchungsbereichen transportiert werden.

Zur Generierung von Magnetfeldern können supraleitende Elektromagnetspulensysteme, resistive Elektromagnetspulensysteme, Permanentmagnetsysteme und/oder eine Kombination daraus genutzt werden.

Eine für den praktischen Einsatz der erfindungsgemäßen Vorrichtung besonders bevorzugte Klasse von Ausführungsformen zeichnet sich dadurch aus, dass das Magnetsystem ein supraleitendes Elektromagnetspulensystem umfasst, welches innerhalb eines Kryostaten angeordnet und im Betrieb supraleitend kurzgeschlossen ist.

Alternativ oder ergänzend kann in einer weiteren Klasse von Ausführungsformen das Magnetsystem ein resistives Elektromagnetspulensystem umfassen, bei welchem alle vorhandenen Teilspulen elektrisch in Serie geschaltet sind.

Im Fall der Nutzung eines oder mehrerer supraleitender Elektromagnetspulensysteme als Feldgenerator ist die Anordnung in Verbindung mit einem einzigen Kryostaten zu realisieren. Supraleitende Elektromagneten ermöglichen die Generierung eines hohen Magnetgrundfeldes B₀ sowie eines starken Magnetfeldgradienten G.

In der MRI wirkt sich die Stärke des Magnetgrundfeldes direkt auf das Signal zu Rausch Verhältnis (SNR) aus, welches näherungsweise proportional mit B₀ ansteigt. In der MPI-Technologie ist das örtliche Auflösungsvermögen für einen bestimmten Tracer proportional zu 1/G. Mit steigendem Magnetfeldgradient schrumpft die Ausdehnung des FFP in dem die Nanopartikel nicht in Sättigung sind und somit zum Empfangssignal beitrage. Im Vergleich zu einer Ausführung mit resistiven Elektromagneten verringert eine Ausführung mit supraleitenden Elektromagneten in Kombination eines Kryostaten den Energiebedarf bei Betrieb sowie die Kühlanforderungen und somit den Platzbedarf innerhalb des Technikraumes drastisch.

Supraleitenden Elektromagneten weisen ferner durch die konstante Temperierung innerhalb des Kryostaten eine hohe Langzeitstabilität auf. Koaxial angeordnete Magnetsysteme erlauben eine einfache Berechnung und Realisierbarkeit der geforderten Magnetfeldhomogenität sowie die des Magnetfeldgradienten.

Bei symmetrisch und koaxial aufgebauten Magnetfeldsystemen ist die Kraftwirkung minimal/symmetrisch und erleichtert somit die Konstruktion.

Eine Ausführung des Feldgenerators mittels eines resistiven Elektromagnetspulensystems, bei welchem alle Teilspulen in Serie geschaltet und koaxial angeordnet sind, ist durch die Vorgabe des Spulenstromes die Höhe des Magnetgrundfeldes sowie die Stärke des Magnetfeldgradienten innerhalb bestimmter Grenzen frei wählbar. Dies ist insbesondere für den MPI-Modus interessant, da die Ausdehnung des field of views (FoV) bei konstanter DF-Amplitude proportional zu 1/G ist.

Resistive Elektromagnetspulensysteme erlauben ferner bei nicht Nutzung des Systems das Abschalten des Grundfeldes/Gradientenfeldes, was die Risiken die mit starken Magnetfeldern einhergehen minimiert. Ferner erlaubt das Abschalten des Grundmagnetfeldes die optimale Nutzung neuer Ansätze der Systemkalibrierung mittels "System Calibration Unit" in der MPI-Bildgebung (siehe Referenz [7]).

Die Nutzung von Permanentmagnetsystemen erlaubt die Generierung von moderaten Magnetgrundfelder sowie starken Magnetfeldgradienten. Die Kühlanforderungen an solche Systeme sind im Vergleich zu resistiven Systemen gering, da diese ausschließlich zur Temperierung der magnetfelderzeugenden Elemente und somit zur Stabilisierung derer magnetischen Eigenschaften nötig ist. Der Einsatz von Permanentmagnetsystemen reduziert zudem den Gesamtenergieverbrauch des Systems bei Betrieb. Zudem kann das Gesamtsystem (inklusive Technikraum) in kompakter Weise realisiert werden.

Für das Positionieren des Untersuchungsobjektes im Zentrum des jeweiligen Untersuchungsbereiches sind Markierungseinrichtungen hilfreich.

Werden Teile dieser Markierungseinrichtungen ferner an einem oder mehreren Raumpunkten der Transporteinheit bzw. Patientenliege und oder dem Messobjekt selbst angebracht und als Protonen-Marker ausgeführt, so können diese Raumpunkte in der MRI-Aufnahme lokalisiert werden. Für eine Raumpunktlokalisation in den MPI-Daten können zusätzlich definierte Raumpunkte mit MPI-Markern versehen werden. Die daraus gewonnenen Informationen der verschiedenen Raumpunkte der jeweiligen Modalität kann zu einer stabilen und präzisen Ko-Registrierung beider Bilddatensätze genutzt werden und erlauben somit eine hohe Genauigkeit der Bildfusion.

Eine optimale Nutzung von allen MRI-Teilkomponenten (wie zum Beispiel die MR-Sende- und/oder Empfangsspule) fordert, dass das homogene magnetische Zentrum (=Isozentrum) des Feldgenerators mit dem magnetischen Zentrum der weiteren MRI-Teilkomponenten bzw. mit dem MRI-Untersuchungsbereich zusammenfällt.

Gleiches gilt für die MPI-Teilkomponenten (wie zum Beispiel die Drive-Field-Spulen), bei denen der Nulldurchgang des Feldbetrages (der FFP) des Feldgenerators mit dem magnetischen Zentrum der weiteren MPI-Teilkomponenten bzw. dem MPI-Untersuchungszentrum zusammenfällt.

Um Interferenzen/Störungen zwischen den MRI-Teilkomponenten und den MPI-Teilkomponenten zu minimieren, müssen beide Untersuchungsbereiche in Bezug auf Hochfrequenz-Strahlung und Niederfrequenz-Strahlung abgeschirmt sein.

Bei bevorzugten Ausführungsformen der Erfindung wird das Streufeld des Magnetsystems durch eine aktive und/oder passive Abschirmung in seiner räumlichen Ausdehnung reduziert.

Um bereits installierte MRI-Geräte zu einem integral aufgebauten MRI-MPI-Hybridgerät aufzurüsten, können magnetfelderzeugende Elemente (resistiv, supraleitend oder permanent) als Anbau/Extension realisiert werden. Eine MPI-Nachrüstung von bestehenden MRI-Geräten wäre im Vergleich zu einer Neuinstallation eines Hybridgerätes eine kostengünstige Alternative mit minimiertem Installationsaufwand durch die Mitnutzung bestehender Komponenten und der umliegenden Infrastruktur.

Eine Realisierung des Feldnulldurchganges des Magnetfeldgradienten als feldfreie Linie (FFL) anstatt eines FFP ermöglicht andere/neue MPI-Akquisitionsansätze mit einer potenziell höheren Sensitivität und kürzere Akquisitionszeit (siehe Referenz [7)].

### Zeichnungen und detaillierte Beschreibung zweier Ausführungsbeispiele

Weitere Vorteile der Erfindung ergeben sich aus der Beschreibung und den Zeichnungen. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung. Die Erfindung ist in den Zeichnungen dargestellt und wird anhand von Ausführungsbeispielen näher erläutert. Es zeigen:
Fig. 1 Schematische Darstellung eines Schnittes durch die magnetfelderzeugenden Elemente 101, 102 eines integral aufgebauten MRI-MPI-Hybridsystems, wobei die Ausführung als resistive und/oder supraleitende Elektromagnetsysteme dargestellt ist. Die Untersuchungsorte für MRI 104 und MPI 105, das Untersuchungsobjekt 103 und die Transporteinheit 106 sind hier schematisch dargestellt.
Fig. 2 Schematischer Schnitt durch die drei axial angeordneten supraleitenden magnetfelderzeugenden Elemente 201, 202, 210 des Ausführungsbeispieis 1 mit Zylindersymmetrie um eine z-Achse. Spulenelement 201 und Spulenelement 202 in einer Maxwell-Konfiguration dienen zur Erzeugung eines Magnetfeldgradienten mit B₀-Feldnulldurchgang zur Bereitstellung eines FFP's 108. Spulenelement 210 dient als aktive Abschirmung des Magnetfeldes nach außen und bewirkt eine Homogenisierung des B₀-Feldes im Inneren.
Fig. 3 Simulation des gesamten axialen Magnetfeldverlaufes bei r=0cm des Ausführungsbeispiels 1 aus Fig. 2. Die erste Region kennzeichnet den Untersuchungsbereich, welcher den MRI-Anforderungen genügt, d.h. ein hohes statisches und homogenes Magnetfeld, wobei z=0 das Iso-Zentrum anzeigt. Die zweite Region kennzeichnet den Untersuchungsbereich, welcher den MPI-Anforderungen genügt, d.h. einen starken Magnetfeldgradienten mit B₀-Nulldurchgang, der den FFP darstellt.

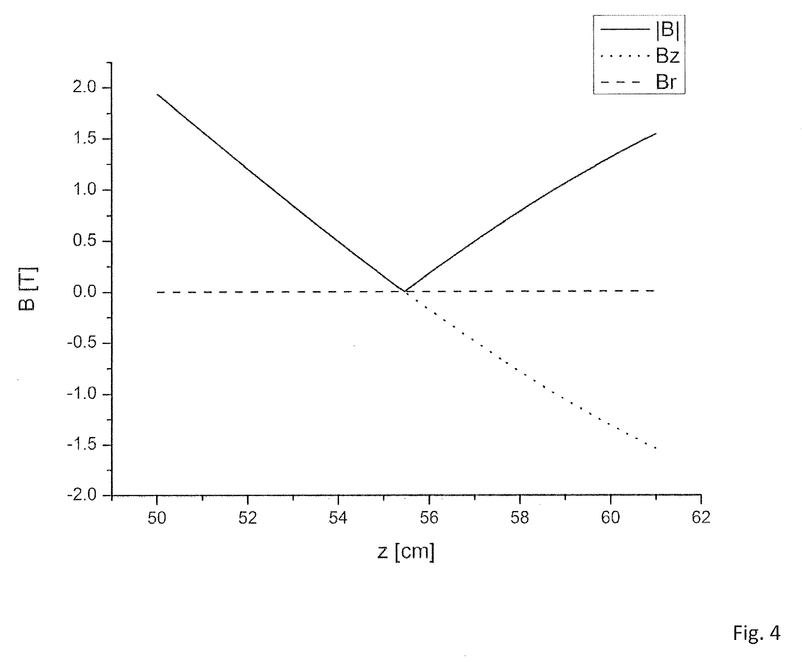

Fig. 4 Simulation des axialen Magnetfeldverlaufes des Ausführungsbeispiels 1 bei r=0cm im Bereich des FFP's des MPI Untersuchungsbereiches.
Fig. 5 Simulation des zylindersymmetrischen Magnetfeldverlaufes des Ausführungsbeispiels 1 in axialer Richtung bei z=55,5cm (FFP) des MPI Untersuchungsbereiches.
Fig. 6 Simulierte Magnetfeldhomogenität des Ausführungsbeispiels 1 im Bereich des Isozentrums 107 des MRI Untersuchungsbereiches.
Fig. 7 Simuliertes Streufeld des Ausführungsbeispiels 1.
Fig. 8 Schnittbild durch den oberen Teil der fünf axial angeordneten Permanentmagnete welche als magnetfelderzeugende Elemente des Ausführungsbeispiels 2 dienen. Die zwei äußeren gegensinnig radial magnetisierten Neodym-Eisen-Bor (NdFeB) Ringe mit einer magnetischen Remanenz von Bᵣ= 1,4T generieren je einen Magnetfeldgradienten mit B₀-Nulldurchgang zur Bereitstellung von zwei FFP sowie einen homogenen Feldbereich B₀. Die mittleren axial magnetisierten Ringelemente dienen zur Homogenisierung des Magnetgrundfeldes. Die Pfeile deuten den Magnetisierungsvektor der NdFeB-Ringe an.
Fig. 9 Simulation des gesamten axialen Magnetfeldverlaufes bei r=0cm des Ausführungsbeispiels 2 aus Fig. 8. Die mittlere Region kennzeichnet den Untersuchungsbereich, welcher den MRI Anforderungen genügt, d.h. ein hohes statisches und homogenes Magnetfeld, wobei das Iso-Zentrum bei z=0 liegt. Die Region um den Nulldurchgang kennzeichnet den Untersuchungsbereich, welcher den MPI Anforderungen genügt, d.h. einen starken Magnetfeldgradienten mit B₀-Nulldurchgang, der den FFP darstellt.
Fig. 10 Simulation des axialen Magnetfeldverfaufes des Ausführungsbeispiels 2 bei r=0cm im Bereich des FFP's (z=16,1cm) des MPI Untersuchungsbereiches.
Fig. 11 Simulation des zylindersymmetrischen Magnetfeldverlaufes des Ausführungsbeispiels 2 in axialer Richtung bei z=16,1cm (FFP) des MPI Untersuchungsbereiches.
Fig. 12 Simulierte Magnetfeldhomogenität des Ausführungsbeispiels 2 im Bereich des Iso-Zentrums des MRI Untersuchungsbereiches.

### Ausführungsbeispiele:

### Ausführungsbeispiel 1 : Supraleitendes Elektromagnetspulensystem

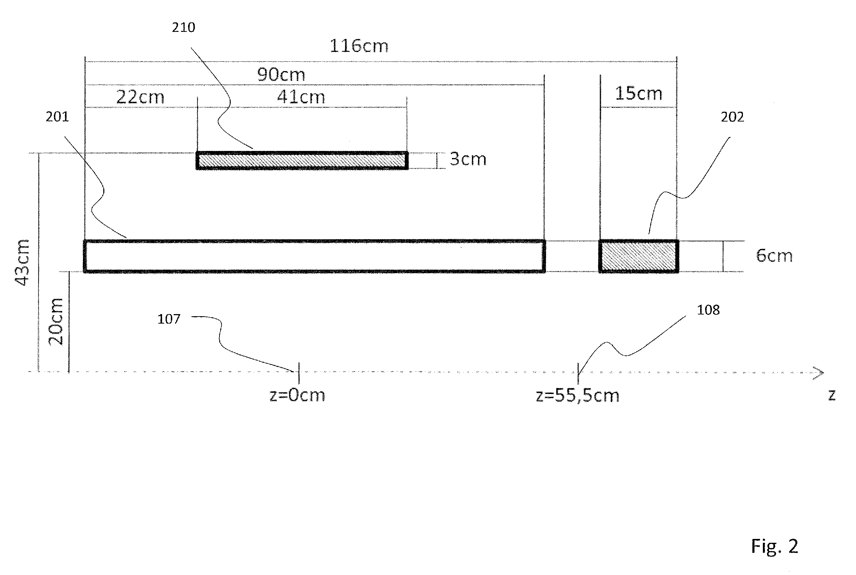

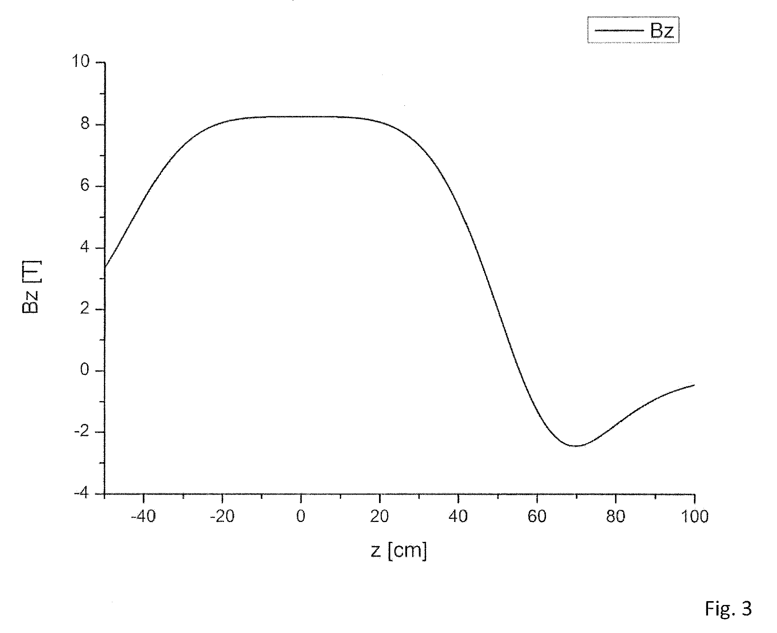

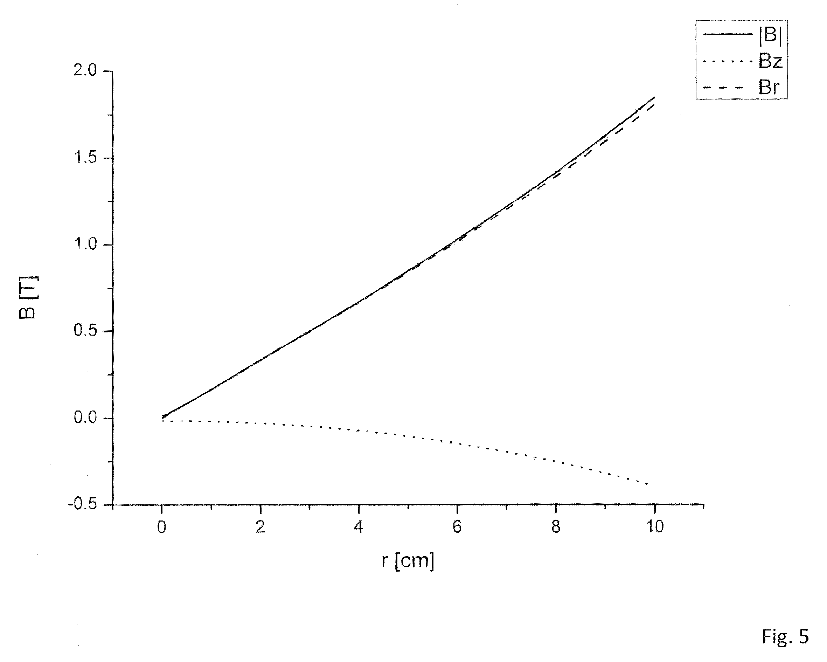

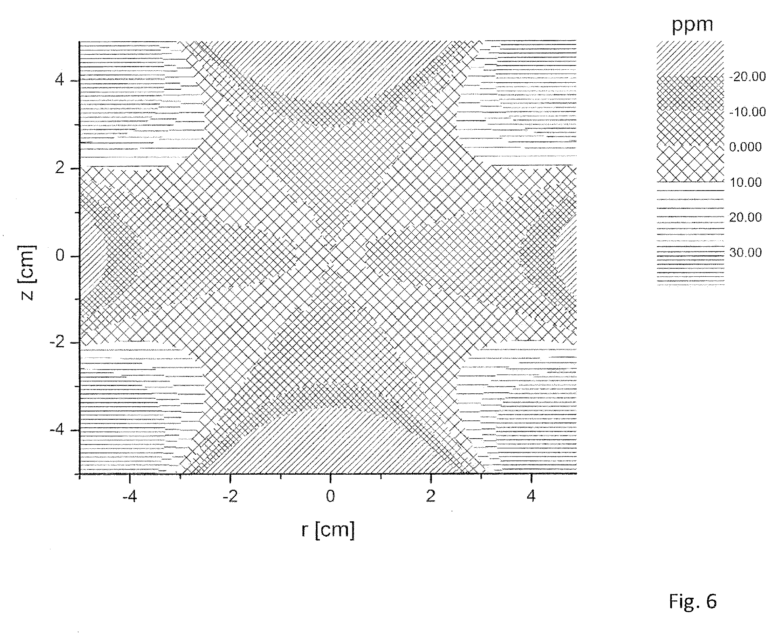

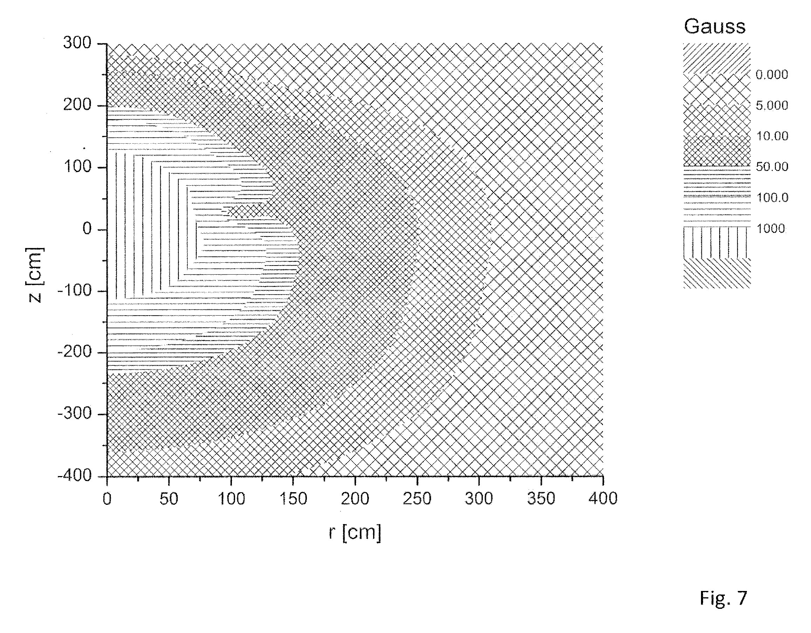

Ausführungsbeispiel 1 nutzt ausschließlich supraleitende magnetfelderzeugende Elemente 201, 202, 210 (siehe Fig. 2), die den zeitlich sich nicht ändernden Magnetfeldverlauf in Fig. 3 generieren. Dieser integrale Aufbau des Hybridsystems besteht aus drei, in einem Kryostaten (4,2K) koaxial angeordneten und aus NbTi-Leiter gewickelten Solenoid-Teilspulenelemente (Gesamtgewicht der Spulen ca. 120kg), dargestellt in Fig. 2. Die Hauptspule 201 erzeugt das Grundmagnetfeld B₀, welches durch Spule 210 im inneren homogenisiert (siehe Fig. 6) und nach außen abgeschirmt wird (siehe Fig. 7). Die Stromflussrichtungen sind bei Spule 201 und 210 antiparallel. Die zur Hauptspule axial verschobene und in Anti-Helmoltzkonfiguration betriebene Spule 202 verstärkt den inhärenten Magnetfeldgradienten der Hauptspule 201 und generiert einen FFP 108 (siehe Fig. 5 und 6). Diese Anordnung liefert ein homogenes Grundmagnetfeld B₀ von ca. 8T im MRI Untersuchungsbereich 104 und einen Magnetfeldgradienten von ca. 35T/m im MPI Untersuchungsbereich 105.

### Ausführungsbeispiel 2: Permanentmagnet

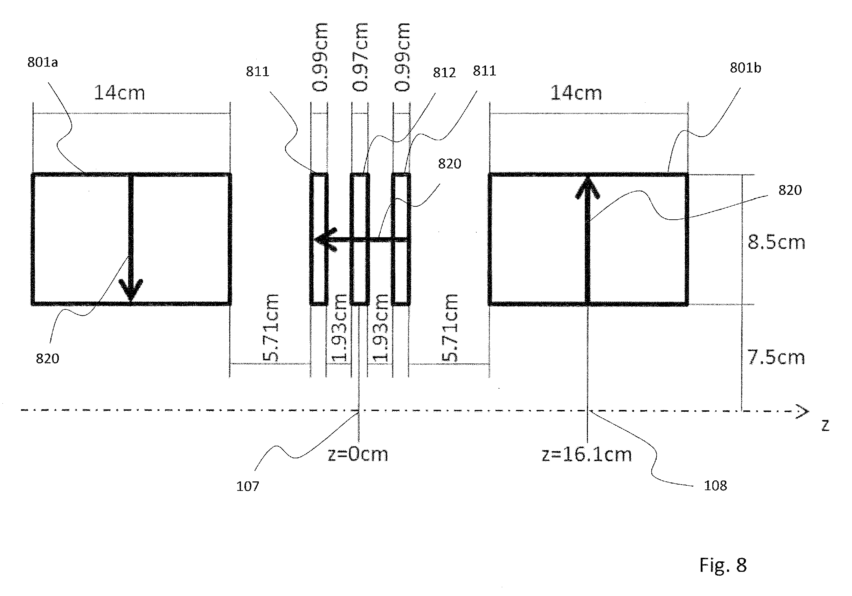

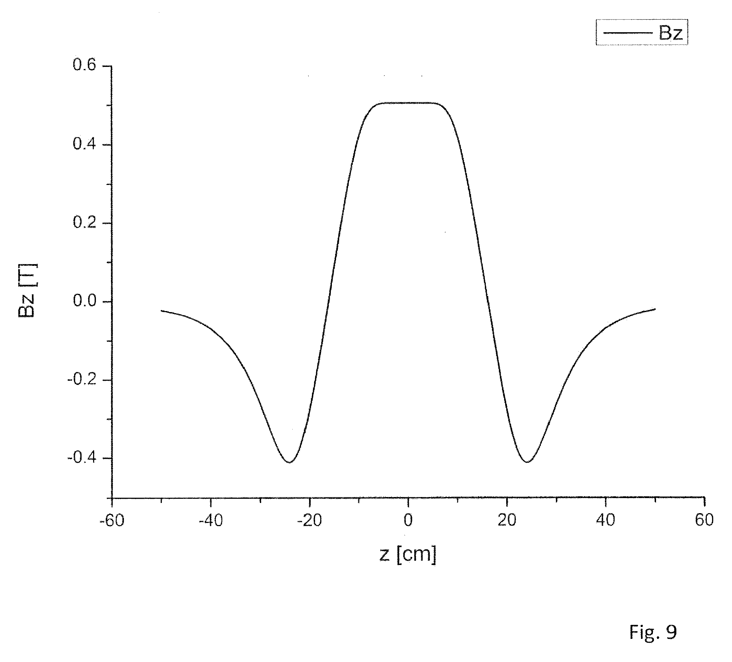

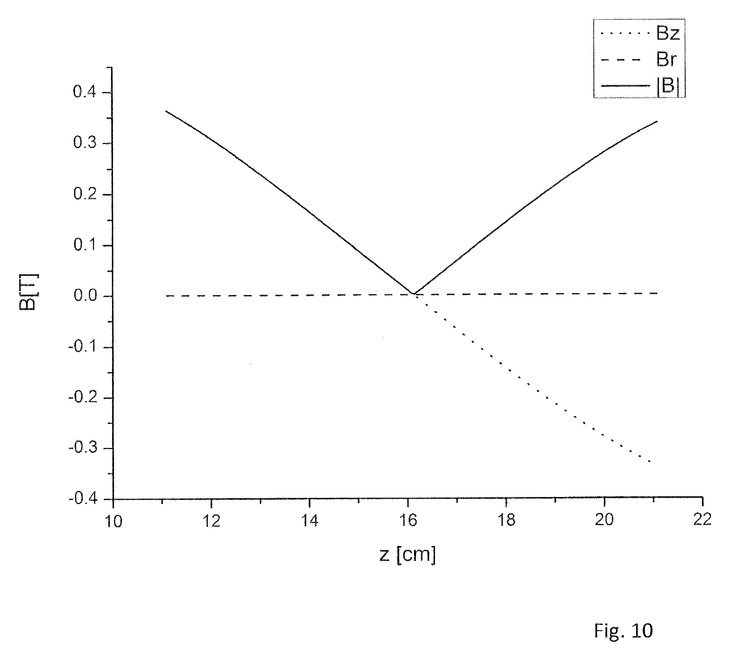

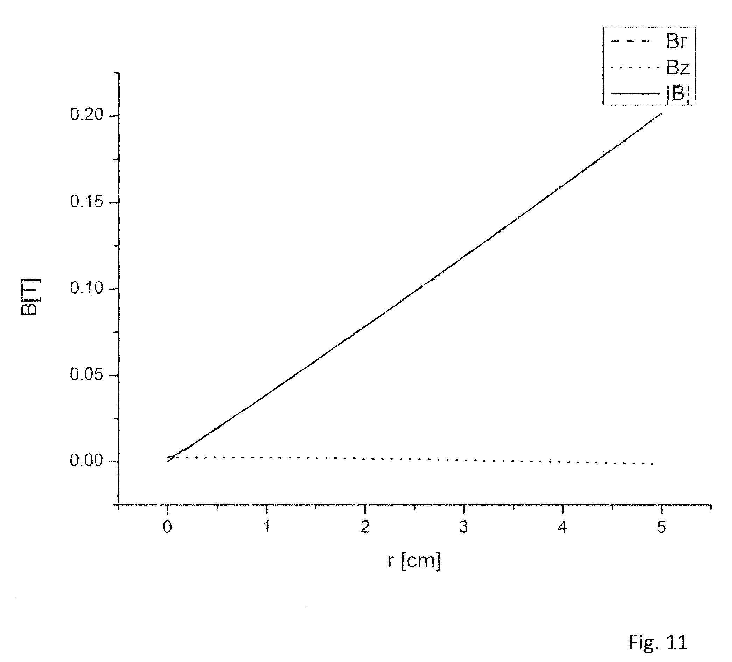

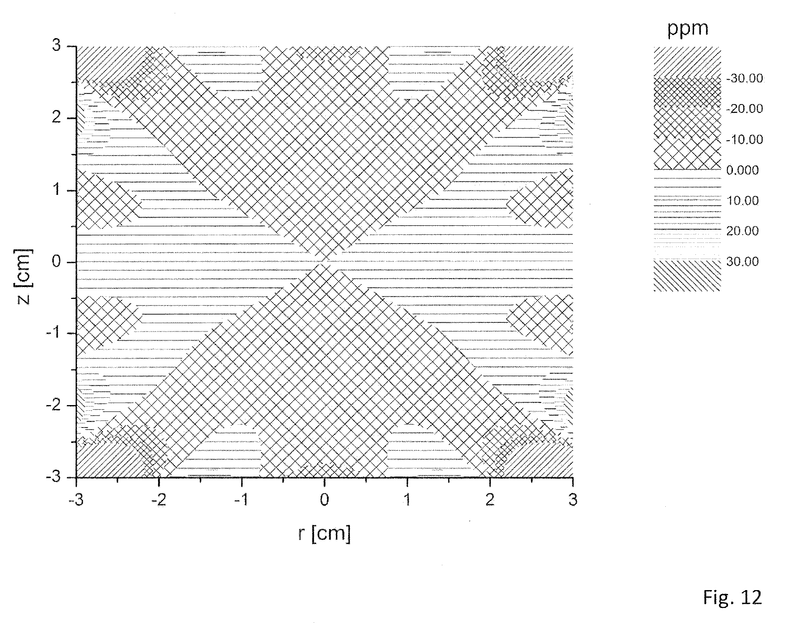

Ausführungsbeispiel 2 nutzt ausschließlich permanente magnetfelderzeugende Elementen 801 a, 801 b, 811, 812, die den zeitlich sich nicht ändernden Magnetfeldverlauf in Fig. 9 generiert. Dieser symmetrische und integrale Aufbau des Hybridsystems besteht aus insgesamt 5 koaxial angeordneten magnetisierten Ringen 801 a, 801 b, 811, 812 (siehe Fig. 8) des Materials Neodym-Eisen-Bor (NdFeB) mit einer magnetischen Remanenz von Br= 1,4T (Gesamtgewicht des Magnetsystems ca. 150kg). Die äußeren Hauptmagnetringe 801 a, 801 b, welche den homogenen Magnetfeld-bereich sowie den Magnetfeldgradienten (siehe Fig. 10-11) generieren, werden in der Praxis aus Segmenten hergestellt um eine radiale Magnetisierungs-richtung zu realisiert. Zur Erhöhung der Magnetfeldhomogenität des B₀-Feldes in der zentralen Region können axial magnetisierten Ringmagneten verwendet werden. In diesem Ausführungsbeispiel werden lediglich drei axial magnetisierte Ringmagnete 811, 812 genutzt, welches einen Kompromiss zwischen Komplexität des Systems und der damit erreichten Feldhomogenität (siehe Fig. 12) darstellt. Durch die in Z-Richtung symmetrische Ausführung entfallen die ungeraden Feldordnungen (Feldverzerrungskomponenten) und erleichtert somit die Homogenisierung des Magnetfeldes in der zentralen Region. Diese Anordnung liefert einen Grundmagnetfeld B₀ von ca. 0,5T im MRI-Untersuchungsbereich 104 und einen Magnetfeldgradienten von ca. 7,7T/m im MPI Untersuchungsbereich 105. Zur weiteren Erhöhung des Magnetfeld-gradienten können die Hauptmagnetringe 801a, 801 b nicht wie in diesem Ausführungsbeispiel zylindrisch sondern konisch (d.h. die Länge der Hauptmagnetringe in Z-Richtung ist am inneren Radius größer als am äußeren Radius) hergestellt werden.

### Bezugszeichenliste

- 101: Solenoid-Hauptspule
- 102: zur Hauptspule in Anti-Helmholzkonfiguration betriebene Solenoid-Spule
- 103: Messobjekt
- 104: MRI-Untersuchungsbereich
- 105: MPI-Untersuchungsbereich
- 106: Transporteinrichtung
- 107: MRI-Isozentrum
- 108: Feldfreier Punkt (MPI-Zentrum)
- 109a: MRI-Marker (Protonen-Marker) an der Liege
- 109a': MRI-Marker (Protonen-Marker) am Messobjekt
- 109b: MPI-Marker an der Liege
- 109b': MPI-Marker am Messobjekt
- 201: supraleitende Solenoid-Hauptspule
- 202: zur Hauptspule in Anti-Helmholzkonfiguration betriebene supraleitende Solenoid-Spule
- 210: zur Hauptspule in Anti-Helmholzkonfiguration betriebene supraleitende Homogenisierungs- und Abschirmungsspule
- 801a: radial magnetisierter Hauptmagnetring
- 801b: radial in Gegenrichtung zu 801amagnetisierter Hauptmagnetring
- 811: axial magnetisierte Ringmagnete für die Homogenisierung des B₀-Feldes
- 812: axial magnetisierter Ringmagnet für die Homogenisierung des B₀-Feldes
- 820: Magnetisierungsrichtung

### Referenzen

[1] Gleich B and Weizenecker J 2005 Tomographic imaging using the nonlinear response of magnetic particles Nature 435 1214-7
[2] Sattel T F, Knopp T, Biederer S, Gleich B, Weizenecker J, Borgert J and Buzug T M 2009 Single-sided device for magnetic particle imaging. J. Phys. D: Appl. Phys. 42 1-5
[3] Weizenecker J, Gleich B, Rahmer J, Dahnke H and Borgert J 2009 Three-dimensional real-time in vivo magnetic particle imaging. Phys. Med. Biol. 54 L1-L10
[4] Weizenecker J, Borgert J and Gleich B 2007 A simulation study on the resolution and sensitivity of magnetic particle imaging. Phys. Med. Biol. 52 6363-74
[5] Knopp T, Biederer S, Sattel T,Weizenecker J, Gleich B, Borgert J and Buzug T M 2009 Trajectory analysis for magnetic particle imaging. Phys. Med. Biol. 54 385-97
[6] Vlaadingerbroek M T and den Boer J A 2003 Magnetic Resonance Imaging. Springer-Verlag 3rd Edition
[7] Magnetic Particle Imaging - A Novl SPIO Nanoparticle Imaging Technique. Springer Springer Proceedings in Physics

## Patentansprüche

1. Vorrichtung zur abwechselnden Untersuchung eines Messobjektes (103) mittels MPI (="Magnetic Particle Imaging") und mittels MRI (="Magnetic Resonance Imaging") innerhalb eines aus einem ersten Magnetfelderzeugenden Element (101; 201) und einem zweiten Magnetfelderzeugenden Element (102; 202) aufgebauten Magnetsystems, wobei das erste Magnetfelderzeugende Element (101; 201) und das zweite Magnetfelderzeugende Element (102; 202) koaxial längs einer z-Achse angeordnete Elektromagnetspulen umfassen, wobei das Magnetsystem einen ersten Untersuchungsbereich (104) für den MRI-Betrieb aufweist, in welchem ein homogenes Magnetfeld erzeugt wird, wobei das Magnetsystem einen zweiten Untersuchungsbereich (105) für den MPI-Betrieb aufweist, in welchem ein räumlich stark variierendes Magnetfeldprofil erzeugt wird, dessen Feldvektoren an allen Raumpunkten bezüglich Richtung und/oder Betrag unterschiedlich sind und an einem Raumpunkt (108) den Feldbetrag Null umfassen, und wobei eine Transporteinrichtung (106) vorgesehen ist, mit der das Messobjekt (103) aus dem ersten Untersuchungsbereich (104) in den zweiten Untersuchungsbereich (105) und/oder umgekehrt bewegt werden kann, **dadurch gekennzeichnet,**
**dass** das erste Magnetfelderzeugende Element (101; 201) als Solenoid-Hauptspule und das zweite Magnetfelderzeugende Element (102; 202) als gegenüber der Solenoid-Hauptspule axial in Richtung der z-Achse verschobene und zur Solenoid-Hauptspule in Anti-Helmholtzkonfiguration betriebene Solenoid-Spule ausgebildet ist,
und **dass** das Magnetsystem ein drittes Magnetfelderzeugendes Element (210) zur Homogenisierung des Magnetfelds im ersten Untersuchungsbereich (104) durch Kompensation des im Untersuchungsbereich (104) aufgrund des zweiten Magnetfeld-erzeugenden Elements (102; 202) verursachten Feldgradienten und zur Abschirmung des Magnetsystems umfasst, wobei das dritte Magnetfelderzeugende Element (210) radial außerhalb des ersten Magnetfelderzeugenden Elements (201) und axial symmetrisch zu einem Isozentrum (107) angeordnet ist, welches an einer axialen Position z=0 im Betrieb der Vorrichtung ein homogenes Magnetfeld radial innerhalb des Magnetsystems aufweist und mit dem magnetischen Zentrum des ersten Untersuchungsbereichs (104) zusammenfällt.

2. Vorrichtung nach Anspruch 1, **dadurch gekennzeichnet, dass** eine die z-Achse senkrecht schneidende gedachte Mittelebene des solenoidförmigen ersten Magnetfeld-erzeugenden Elements (201) gegenüber der axialen Position z=0 in Richtung auf das zweite Magnetfelderzeugende Element (202) hin verschoben angeordnet ist.

3. Vorrichtung nach Anspruch 1 oder 2, **dadurch gekennzeichnet, dass** mindestens eines der Magnetfelderzeugenden Elemente ein Permanentmagnet ist.

4. Vorrichtung nach einem der vorhergehenden Ansprüche, **dadurch gekennzeichnet, dass** das Magnetsystem ein Elektromagnetspulensystem umfasst, bei welchem alle vorhandenen Teilspulen elektrisch in Serie geschaltet sind.

5. Vorrichtung nach einem der vorhergehenden Ansprüche, **dadurch gekennzeichnet**, das Magnetsystem ein supraleitendes Elektromagnetspulensystem umfasst, welches innerhalb eines Kryostaten angeordnet und im Betrieb supraleitend kurzgeschlossen ist.

6. Vorrichtung nach einem der vorhergehenden Ansprüche, **dadurch gekennzeichnet, dass** das erste Magnetfelderzeugende Element (101; 201) und das zweite Magnetfelderzeugende Element (102; 202) des Magnetsystems in einem gemeinsamen Gehäuse angeordnet sind.

7. Vorrichtung nach einem der vorhergehenden Ansprüche, **dadurch gekennzeichnet, dass** eine Markierungseinrichtung mindestens einen Protonen-Marker (109a, 109a') zur Hervorhebung eines oder mehrerer Raumpunkte des ersten Untersuchungsbereichs (104) auf einer MRI-Aufnahme und/oder mindestens einen MPI-Marker (109b, 109b') zur Hervorhebung eines oder mehrerer Raumpunkte des zweiten Untersuchungsbereichs (105) auf einer MPI-Aufnahme umfasst.

8. Vorrichtung nach Anspruch 7, **dadurch gekennzeichnet, dass** zumindest Teile der Markierungseinrichtung an einer Liege (106) für das Messobjekt (103) bzw. einen mit der Vorrichtung zu untersuchenden Patienten angebracht sind.

9. Vorrichtung nach einem der vorhergehenden Ansprüche, **dadurch gekennzeichnet, dass** der erste Untersuchungsbereich (104) und der zweite Untersuchungsbereich (105) sowohl bezüglich Hochfrequenz-Strahlung als auch bezüglich Niederfrequenz-Strahlung abgeschirmt sind.

10. Vorrichtung nach einem der vorhergehenden Ansprüche, **dadurch gekennzeichnet, dass** der Nulldurchgang des Feldgradienten eine Feldfreie Linie ist.

11. Vorrichtung nach einem der vorgehenden Ansprüche, **dadurch gekennzeichnet, dass** das Streufeld des Magnetsystems durch eine aktive und/oder passive Abschirmung in seiner räumlichen Ausdehnung reduziert wird.

## Claims

1. Device for alternating examination of a measurement object (103) by means of MPI (="Magnetic Particle Imaging") and MRI (="Magnetic Resonance Imaging") within a magnet system comprised of a first magnetic field generating element (101;201) and a second magnetic field generating element (102;202), wherein the first magnetic field generating element (101;201) and the second magnetic field generating element (102;202) comprise electromagnetic coils disposed coaxially along a z-axis, wherein the magnet system has a first examination region (104) for the MRI operation, in which a homogenous magnetic field is generated, wherein the magnet system has a second examination region (105) for the MPI operation, in which a spatially strongly varying magnetic field profile is generated, the field vectors thereof being different at all spatial points with regard to direction and/or amount and having one spatial point (108) of zero field magnitude, the device having a transport apparatus (106) by means of which the measurement object (103) can be moved out of the first examination region (104) and into the second examination region (105) and/or vice versa **characterized in that**,
the first magnetic field generating element (101;201) is designed to be a main solenoid coil and the second magnetic field generating element (102;202) is designed to be a solenoid coil which is displaced axially in the direction of the z-axis relative to the main solenoid coil and which is operated in anti-Helmholtz configuration with respect to the main solenoid coil,
and that the magnet system comprises a third magnetic field generating element (210) for homogenizing the magnetic field in the first examination region (104) by compensating for the field gradient generated by the second magnetic field generating element (102;202) in the examination region (104) and for shielding the magnet system, wherein the third magnetic field generating element (210) is disposed radially outside of the first magnetic field generating element (201) and axially symmetric relative to an isocenter (107), which, during operation of the device, has a homogeneous magnetic field radially within the magnet system at an axial position z=0 and which coincides with the magnetic center of the first examination region (104).

2. Device according to claim 1, **characterized in that** a conceptual middle plane of the solenoidal first magnetic field generating element (201), which is perpendicular to and intersects the z-axis, is positioned displaced away from the z=0 axial position towards the second magnetic field generating element (202).

3. Device according to claim 1 or 2, **characterized in that** at least one of the magnetic field generating elements is a permanent magnet.

4. Device according to any one of the preceding claims, **characterized in that** the magnet system comprises an electromagnetic coil system, in which all of its partial coils are electrically connected in series.

5. Device according to any one of the preceding claims, **characterized in that** the magnet system comprises a superconducting electromagnetic coil system, which is disposed within a cryostat and is superconductively short-circuited during operation.

6. Device according to any one of the previous claims, **characterized in that** the first magnetic field generating element (101;201) and the second magnetic field generating element (102;202) of the magnet system are disposed in a common housing.

7. Device according to any one of the previous claims, **characterized in that** a marking device comprises at least one proton marker (109a, 109a') to highlight one or more spatial points of the first examination region (104) on an MRI image and/or at least one MPI marker (109b, 109b') to highlight one or more spatial points of the second examination region (105) on an MPI image.

8. Device according to claim 7, **characterized in that** at least parts of the marking device are mounted on a table (106) for the measurement object (103) or for the patient to be examined with the device.

9. Device according to any one of the previous claims, **characterized in that** the first examination region (104) and the second examination region (105) are shielded with respect to both radio-frequency radiation and low-frequency radiation.

10. Device according to any one of the previous claims, **characterized in that** the zero crossing of the field gradient is a field-free line.

11. Device according to any one of the previous claims, **characterized in that** the stray field of the magnet system is reduced in its spatial extent by an active and/or passive shielding.

## Revendications

1. Dispositif permettant d'examiner alternativement un objet à mesurer (103) par IPM (= « imagerie à particules magnétiques ») et par IRM (= « imagerie par résonance magnétique ») à l'intérieur d'un système magnétique constitué d'un premier élément générateur de champ magnétique (101 ; 201) et d'un deuxième élément générateur de champ magnétique (102 ; 202), le premier élément générateur de champ magnétique (101 ; 201) et le deuxième élément générateur de champ magnétique (102 ; 202) comprenant des bobines électromagnétiques disposées coaxialement le long d'un axe z, le système magnétique présentant une première zone d'examen (104) pour le mode IRM, dans laquelle un champ magnétique homogène est généré, le système magnétique présentant une deuxième zone d'examen (105) pour le mode IPM, dans laquelle est généré un profil de champ magnétique fortement variable spatialement, dont les vecteurs de champ sont différents en ce qui concerne la direction et/ou la grandeur à tous les points de l'espace et comprennent une grandeur de champ nulle à un point de l'espace (108), et un dispositif de transport (106) étant prévu, qui permet de déplacer l'objet à mesurer (103) de la première zone d'examen (104) dans la deuxième zone d'examen (105) et/ou inversement,
**caractérisé en ce**
**que** le premier élément générateur de champ magnétique (101 ; 201) est réalisé sous la forme d'une bobine principale de solénoïde et le deuxième élément générateur de champ magnétique (102 ; 202) sous la forme d'une bobine de solénoïde décalée axialement dans la direction de l'axe z par rapport à la bobine principale de solénoïde et fonctionnant en configuration anti-Helmholtz par rapport à la bobine principale de solénoïde,
et **que** le système magnétique comprend un troisième élément générateur de champ magnétique (210) pour homogénéiser le champ magnétique dans la première zone d'examen (104) par compensation du gradient de champ provoqué dans la zone d'examen (104) par le deuxième élément générateur de champ magnétique (102 ; 202) et pour blinder le système magnétique, le troisième élément générateur de champ magnétique (210) étant disposé radialement à l'extérieur du premier élément générateur de champ magnétique (201) et de façon symétrique axialement par rapport à un isocentre (107) qui présente un champ magnétique homogène radialement à l'intérieur du système magnétique à une position axiale z=0 pendant le fonctionnement du dispositif et qui coïncide avec le centre magnétique de la première zone d'examen (104).

2. Dispositif selon la revendication 1, **caractérisé en ce qu'**un plan médian imaginaire du premier élément générateur de champ magnétique (201) en forme de solénoïde qui coupe l'axe z perpendiculairement est décalé en direction du deuxième élément générateur de champ magnétique (202) par rapport à la position axiale z=0.

3. Dispositif selon la revendication 1 ou 2, **caractérisé en ce qu'**au moins un des éléments générateurs de champ magnétique est un aimant permanent.

4. Dispositif selon l'une des revendications précédentes, **caractérisé en ce que** le système magnétique comprend un système de bobines électromagnétiques dans lequel toutes les bobines partielles présentes sont couplées en série électriquement.

5. Dispositif selon l'une des revendications précédentes, **caractérisé en ce que** le système magnétique comprend un système de bobines électromagnétiques supraconducteur qui est disposé à l'intérieur d'un cryostat et court-circuité de manière supraconductrice en fonctionnement.

6. Dispositif selon l'une des revendications précédentes, **caractérisé en ce que** le premier élément générateur de champ magnétique (101 ; 201) et le deuxième élément générateur de champ magnétique (102 ; 202) du système magnétique sont disposés dans un boîtier commun.

7. Dispositif selon l'une des revendications précédentes, **caractérisé en ce qu'**un dispositif de marquage comprend au moins un marqueur de protons (109a, 109a') pour faire ressortir un ou plusieurs points de l'espace de la première zone d'examen (104) sur une image d'IRM et/ou au moins un marqueur IPM (109b, 109b') pour faire ressortir un ou plusieurs points de l'espace de la deuxième zone d'examen (105) sur une image d'IPM.

8. Dispositif selon la revendication 7, **caractérisé en ce qu'**au moins des parties du dispositif de marquage sont montées sur un lit (106) pour l'objet à mesurer (103) ou un patient à examiner avec le dispositif.

9. Dispositif selon l'une des revendications précédentes, **caractérisé en ce que** la première zone d'examen (104) et la deuxième zone d'examen (105) sont blindées aussi bien en ce qui concerne le rayonnement à haute fréquence qu'en ce qui concerne le rayonnement à basse fréquence.

10. Dispositif selon l'une des revendications précédentes, **caractérisé en ce que** le passage par zéro du gradient de champ est une ligne exempte de champ.

11. Dispositif selon l'une des revendications précédentes, **caractérisé en ce que** le champ de dispersion du système magnétique est réduit dans son extension spatiale par un blindage actif et/ou passif.
